# EUROPEAN PATENT APPLICATION

(11) **EP 3 995 080 A1**
(43) Date of publication of application: **11.05.2022**
(21) Application number: 20206384.8
(22) Date of filing: 09.11.2020
(51) Int. Cl.: A61B 6/00, H05G 1/54, G16H 40/40

(54) **MAINTENANCE PREDICTION FOR A MEDICAL IMAGING APPARATUS**

(71) Applicant: Koninklijke Philips N.V., 5656 AG Eindhoven (NL)
(72) Inventor: VOGTMEIER, Gereon, 5656 AE Eindhoven (NL)
(74) Representative: Philips Intellectual Property & Standards

(57) **Abstract**

The invention concerns a monitoring device for maintenance prediction for a medical imaging apparatus, a medical imaging apparatus comprising a monitoring device and a method of maintenance prediction for a medical imaging apparatus. The method comprises the steps of measuring a first data set of at least one parameter of the medical imaging apparatus by at least one sensor, wherein the measuring of the first data set is performed over a predetermined first period of measurement time. Analysing , by a data processing unit, the first data set thereby determining a plurality of operation modes, in which the medical imaging apparatus was during the measurement time, based on the measured parameter, and measuring at least a second data set of the at least one parameter by the at least one sensor, wherein the measuring of the second data set is performed over a predetermined second period of measurement time. Further, comprises the step of analysing, by the data processing unit, the second data set thereby determining at least one operation modus, in which the medical imaging apparatus was during the second measurement time, and comparing the operation modus determined from the second data set with the plurality of operation modes of the first data set by the data processing unit thereby identifying from the plurality of operation modes of the first data set the operation modus which corresponds to the operation modus determined from the second data set by the data processing unit. Further, validating, by the data processing unit, whether values of the measured parameter of the first and second data set differ from each other, and generating a feedback signal based on the result of the validation by the data processing unit, wherein the feedback signal is embodied as a maintenance prediction information for a user of the medical imaging apparatus.

## Description

### FIELD OF THE INVENTION

The invention relates to the field of maintenance prediction for a medical imaging apparatus, and more specifically to a method of maintenance prediction for the medical imaging apparatus, a medical imaging apparatus configured for performing the method, and a computer program element for causing a monitoring device to performing the method of maintenance prediction.

### BACKGROUND OF THE INVENTION

Monitoring basic functionalities of a medical imaging apparatus may be necessary a for the prediction maintenance of the medical imaging apparatus. Especially degradation and/or changes in performance of the medical imaging apparatus and its components may be monitored which changes may influence a proper operation of the medical imaging apparatus. Sensors may be used for supervising several components of the medical imaging device. However, these sensors and their respective signals may be influenced by an actual operation mode and the short term operation history of the medical imaging apparatus. Changes may be measured by direct and/or indirect effects on the medical imaging apparatus. In particular, a medical imaging apparatus is operated under several different conditions like hospital operation modes, temperatures, acquisition modes and sequences with breaks which result in many different effects for, for example the mechanical forces and the operations conditions of the medical imaging apparatus. Because of the influence of the different operation modes onto the sensors, the analysis of the acquired data comprises some special challenges, in particular which data is relevant and should be taken into account for a maintenance prediction.

### SUMMARY OF THE INVENTION

Therefore, there exist a need for optimizing a maintenance prediction for a medical imaging apparatus. In particular, there exist a need for being able to improve an analysis of acquired data and for being able to separate non-relevant data and take only into account relevant data for a maintenance analysis.

An object of the invention is to provide a method for maintenance prediction, a monitoring device for maintenance prediction, and a medical imaging apparatus comprising the monitoring device for maintenance prediction of the medical imaging apparatus during an operation of the medical imaging apparatus. Based on the method, the measured data, and the respective analysis of the data a maintenance prediction can be improved.

The object of the present invention is solved with the subject matter of the independent claims, wherein further embodiments are incorporated in the dependent claims.

According to a first aspect of the present invention, a method of maintenance prediction used for a medical imaging apparatus is described. The method comprises the steps of measuring a first data set of at least one parameter of the medical imaging apparatus by at least one sensor. The measuring of the first data set is performed over a predetermined first period of measurement time. The method additionally comprises analyzing, by a data processing unit, the first data set thereby determining a plurality of operation modes, in which the medical imaging apparatus was during the measurement time, based on the measured parameter. The method further comprises the step of measuring at least a second data set of the at least one parameter by the at least one sensor, wherein the measuring of the second data set is performed over a predetermined second period of measurement time. Further, the method comprises the step of analyzing, by the data processing unit, the second data set thereby determining at least one operation modus, in which the medical imaging apparatus was during the second measurement time. Further the method comprises, the step of comparing the operation modus determined from the second data set with the plurality of operation modes of the first data set by the data processing unit thereby identifying from the plurality of operation modes of the first data set the operation modus which corresponds to the operation modus determined from the second data set by the data processing unit. Furthermore, the method comprises validating, by the data processing unit, whether values of the measured parameter of the first and second data set differ from each other, and generating a feedback signal based on the result of the validation by the data processing unit, wherein the feedback signal is embodied as a maintenance prediction information for a user of the medical imaging apparatus.

In the context of the present invention, the term "maintenance prediction" shall be understood to describe, a statement about the lifetime of a medical imaging apparatus and/or a component, which should be repaired or replaced. The maintenance prediction may be understood to describe an estimation of a status indicating a duration, for example a time, how long the apparatus, component and/or device may still operate properly or when a failure may occur which may cause a replacement or repair of the apparatus, component and/or device.

In the context of the present invention, the term "medical imaging apparatus" shall be understood to describe any kind of medical apparatus suitable to perform imaging of a patient and able to generate a medical image of a patient or body parts of the patient. In particular, the medical imaging apparatus may not be limited to any specific medical imaging process. The described examples and embodiments herein below may be described in reference to an X-ray imaging device, in particular an X-ray tube, wherein this may not be understood limiting the application of the monitoring device. In addition, other medical imaging apparatuses may be monitored by the monitoring device. Further, as is appreciated by the skilled reader the term medical imaging apparatus in general may refer to the apparatus itself, but also the term components of the medical imaging apparatus may equivalently be used and shall be used for the description of the respective embodiment.

In the context of the present invention, the term "parameter of the medical imaging apparatus" shall be understood to describe a measurable physical value, which may influence the functionality, e.g. the operation, of the medical imaging apparatus. The parameter may be any kind of electrical, mechanical, magnetic, optical parameter, which can be measured at the medical imaging apparatus. Further, the measured parameter may not only describe one value it may also be used to describe a range of the value or a specific pattern of the value.

In the context of the present invention, the term "period of measurement time" shall be understood to describe a duration during which the parameter is measured by the sensor, wherein the duration may depend on predetermined settings or on the parameter itself and can be chosen by the user accordingly and using the present disclosure, which will be explained in more detail in the respective embodiments. For instance, the period may be repeated daily and/or repeated every image acquisition and/or every CT-scan.

In the context of the present invention, the term "operation modus" shall be understood to describe a modus of the medical imaging apparatus, and/or of the component of the medical imaging apparatus, during the operation of the medical imaging apparatus (of the component). The medical imaging apparatus may be operated in several different conditions, which may occur periodically, or may be equal over time for a respective parameter. For instance, these conditions may be an operation temperature, a hospital operation mode, day and night operation cycles, continuous data acquisition mode, sequential data acquisition mode. Moreover, the operation mode may be a specific temperature range in which the medical imaging apparatus generally operates, e.g. 15°C to 30°C. Optionally, an operation mode may be the rotation of an X-ray tube in a CT-gantry. In specific angle positions (0°, 90°, and other) during the rotation of the X-ray tube in the CT-gantry different forces apply to the X-ray tube and its components, for example onto the bearing, such that the operation mode of rotation may be further divided into operation mode of rotation and respective angle positions. Other examples of operation mode may be the operation parameter of the electron beam on the X-ray tube, wherein this parameter may further be separated into a respective electron focus of the electron beam. Accordingly, the operation mode referring to the rotation may be further separated into a rotation of the X-ray tube and the different angle positions. Therefore, the operation modes may not only depend on at least one parameter, or may be defined by at least one parameter but also by different, a plurality of parameters which would later allow the tracking of sensor data changes over the period of measurement time for the individual operation mode. More examples of operation modes may be a full load operation, a half load operation of the medical imaging apparatus, angle position of a rotating component of the medical imaging apparatus, a continuous operation of the medical imaging apparatus over a predetermined period of time (one day, one week), a scanning time of the medical imaging apparatus. In particular, the operation mode may be a periodically pattern of a measured parameter during the respective operation mode.

In other words, the described method is used for data acquisition of at least two different data sets, which should be compared with each other, wherein each data set comprises information about a specific operation modus in which the medical imaging apparatus was during the period of measurement time. Further, the data sets are validated whether the measured parameter included in the data set differ from each other. For instance, this may include identification of matching data set and/or data samples from the first data set and the second data set (and/or more data sets). Optionally, this may comprise the identification of differences between the measured data, whether comparable signals in a predetermined tolerance range are included in the data, and may help to identify a drift of signals over time and/or a change of signals due to events. The method and respective monitoring devices using the method presented herein may be used to monitor the medical imaging apparatus directly, which means, wherein the method is used to receive respective signals from the medical imaging apparatus. On the other hand, the method may be used to monitor a specific component and/or specific components of the medical imaging apparatus, such that at least a part of the medical imaging apparatus is analyzed. Therefore, it may not be necessary to analyses all parameters, which means not all components, e.g. the whole medical imaging apparatus, but only some of the parameters or parts of the medical imaging apparatus. By applying the method to the medical imaging apparatus or components thereof, changes in performance and degradation of the medical imaging apparatus or the component can be measured and determined. Further, a lifetime of the medical imaging apparatus and/or its component can be predicted and additionally a prediction of maintenance may be made, such that the medical imaging apparatus or components thereof may be repaired or changed before an end of the lifetime. Depending on the amount of parameters, the maintenance prediction may be improved, such that more parameter allow a more precise estimation of maintenance.

As the data processing unit, a processor may be used, wherein the processor may detect the continuous sensor data and filter it with respect to the identified operation modes to store only the identified data set or data samples. In particular, only the identified and specific data sets may be taken into account for the lifetime, e.g. the maintenance prediction, This may give much more comparable settings and deviations from the standard values as changes of the signal intensity, changes in the spectral pattern and other changes can be detected in a more specific setting and the sensitivity of the method may be increased. Additionally, the changes and effects onto the medical imaging apparatus can be correlated much better with the individual operation modes and via this also with the unique failure mechanisms.

According to an exemplary embodiment of the invention, the measured parameter may be at least one of a vibration of a component of the medical imaging apparatus, a vibration of the medical imaging apparatus, an acceleration of a component of the medical imaging apparatus, an acceleration of the medical imaging apparatus, a surrounding temperature of a component of the medical imaging apparatus, a surrounding temperature of the medical imaging apparatus, a surrounding humidity of a component of the medical imaging apparatus, a surrounding humidity of the medical imaging apparatus, a temperature of the medical imaging apparatus, a temperature of a component of the medical imaging apparatus, a mechanical shock of the medical imaging apparatus, a mechanical shock of a component of the medical imaging apparatus, a vacuum parameter of the medical imaging apparatus, an emitter parameter of the medical imaging apparatus, a power supply parameter of the medical imaging apparatus, an over/under voltage parameter of a component of the medical imaging apparatus, an over/under voltage parameter of the medical imaging apparatus, an over/under current parameter of the medical imaging apparatus, a X-ray radiation parameter of the medical imaging apparatus, a rotation speed of a component of the medical imaging of the apparatus relative to the medical imaging apparatus, a rotation speed of the medical imaging apparatus, an electron beam parameter of the medical imaging apparatus, and a magnetic field parameter of the medical imaging apparatus.

This listing may not be limiting, other parameters may also be measured. For instance, at least all relevant physical parameters of at least a component of the medical imaging apparatus and/or of the medical imaging apparatus itself may be measured. The sensor may be configured for measuring, an instability parameter of the medical imaging apparatus, and/or disturbing signals disturbing the measurement of the sensor. Disturbing signals may be any signals from surrounding electronically devices, which may influence the measured parameter signal, wherein when measuring the disturbing signals these may be filtered and thereby excluded from the interesting signal of the medical imaging apparatus.

The vibration of the component and/or the medical imaging apparatus may be measured using acceleration sensors, for instance the vibration of the bearing in a rotating system may be measured. The vibration may be any kind of mechanical vibration caused by the medical imaging apparatus itself or by the environment of the medical imaging apparatus.

The acceleration of the component and/or the medical imaging apparatus may be also measured by acceleration sensors. For instance, the acceleration may be the chosen parameter to be measured during the phase of transport and storage, wherein the acceleration may be an acceleration with respect to the ground above which the medical imaging apparatus may be positioned or transported, or it may be an acceleration with respect to a medical imaging system in which the medical imaging apparatus is integrated, and/or in which the component is integrated. For instance, an acceleration may be measured when the X-ray tube may fall to ground during transport, or when it is operated in a rotating CT-gantry.

The surrounding temperature of the component and/or the medical imaging apparatus may be the temperature of the direct surrounding in which the medical imaging apparatus is located.

The surrounding humidity of the component and/or the medical imaging apparatus may be the humidity of the direct surrounding in which the medical imaging apparatus is located.

The temperature of the component and/or the medical imaging apparatus may be preferably measured during the phase of operation. The temperature may be a temperature of the housing which should not exceed a surrounding temperature at normal conditions, for instance between 15°C to 40 °C. Further, it may be a temperature of cooling for the medical imaging apparatus, of a motor oil used in a motor of the medical imaging apparatus or used in any pneumatic parts of the medical imaging apparatus, a cooling oil for an X-ray tube, wherein the cooling oil is the cooling liquid. Moreover, this parameter may be transport or storage temperatures which should be detected and which may indicate an influence of the functionality, for the respective phases, may be even down - 20 or - 40°C or up to + 50°C or more (which may occur when the apparatus is transported, stored in an airplane in desert, or shipment in a container).

The mechanical shock of the component and/or the medical imaging apparatus may be any kind of sudden acceleration caused for example by impact, drop, kick or the like. Mechanical shock describes matter subject to extreme rates of force with respect to time. A shock pulse can be characterized by its peak acceleration, the duration, and the shape of the shock pulse (half sine, triangular, trapezoidal, etc.), wherein a shock response spectrum may be a method for further evaluating a mechanical shock.

The vacuum parameter of the component and/or the medical imaging apparatus may be preferably measured during the phase of operation.

The emitter parameter of the component and/or the medical imaging apparatus may be preferably measured during the phase of operation. The emitter parameter may be an emitter voltage, an emitter current used for generating an X-ray beam, an electron beam or the like.

The over/under voltage parameter of the component and/or the medical imaging apparatus may be preferably measured during the phase of operation, wherein this parameter may be a high voltage parameter of a high voltage unit of an X-ray tube. Any variation of high voltage, in combination with (too) high current, may influence stress of the X-ray tube. The over/under voltage parameter may be measured for indicating any electrical short cuts influencing the medical imaging apparatus, for example arcing events may be detected or even prevented, which may influence the vacuum quality.

The over/under current parameter of the component and/or the medical imaging apparatus may be preferably measured during the phase of operation.

The X-ray radiation parameter of the component and/or the medical imaging apparatus may be any change in the X-ray radiation parameter. For instance, this parameter may be an intensity of the X-ray radiation and a variation of the intensity of the X-ray radiation. Further, it may be a radiation spectrum of the component and/or the medical imaging apparatus may be a change in the radiation spectra, and spectral change of energy spectrum of X-ray radiation. A change in the spectrum may indicate a change in the operation condition and can give early indications in potential failure modes.

The rotation speed of the component and/or the medical imaging apparatus may be preferably measured during the phase of operation, wherein the rotation speed may be the rotation speed of the component with respect to the medical imaging apparatus or the rotation speed of the medical imaging apparatus with respect to a medical imaging system.

The electron beam parameter of the component and/or the medical imaging apparatus may be preferably measured during the phase of operation, wherein this parameter may be the focus of the electron beam.

The magnetic field parameter of the component and/or the medical imaging apparatus may be preferably measured during the phase of operation, wherein the magnetic field parameter may be any kind of magnetic field parameter of an X-ray tube, e.g. beam steering, or motor rotation effects, or a magnetic field parameter of an MRI apparatus as the medical imaging apparatus.

Moreover, the sensor may be configured for measuring at least one digital signal of the data processing unit by at least one sensor thereby determining a status of the data processing of the data processing unit. For instance, the measurement of a digital signal may refer to a measurement of a control signal used for controlling the sensor and /or the data acquisition, hence the data processing unit. For preventing failures of data transmission and data processing, these digital signals may be measured and monitored, such that a replacement and/or repair of the component generating a digital signal can be predicted.

According to an exemplary embodiment of the invention, the method may further comprise selecting data samples, at one or more predefined trigger points, of the second data set. The step of analyzing the second data set may further comprise only the analysis of the selected data samples, and comparing, by the data processing unit, the data samples selected from the second data set with the plurality of operation modes of the first data set thereby identifying, by the data processing unit, from the plurality of operation modes of the first data set the operation modus which corresponds to the data samples determined from the second data set. Further, wherein the step of validating comprises validating whether values of the measured parameter of the compared data samples of the second data set and the operation modes of the first data set differ from each other. In other words, not the complete measured data set may analyzed, instead only sections, e.g. snap shots, thereof may be analyzed. The predefined trigger point may be a start point for the data acquisition, or may be a range of the parameter in which the data acquisition should be carried out. For instance, the data set comprises a measured parameter of temperature in a range of 10°C to 30°C, wherein as relevant data samples only the temperature range between 20°C to 25°C is relevant, such that only this range may be analyzed and stored.

According to an exemplary embodiment of the invention, the method may further comprise that the predetermined first period of measurement time corresponds to an active operating life of the medical imaging apparatus, wherein the active operating life of the medical imaging apparatus is one week of active operation life of the medical imaging apparatus. The active operation life shall be understood to describe the time during which the medical imaging apparatus and/or the component thereof is actively operating, such that the active operating life excludes breaks in which the medical imaging apparatus is not working. The active operation life may also be only days, weeks, month but also dedicated operation cases with similar usage pattern that might happen in an unregularly time distance which can be chosen by the user accordingly and in dependency of the parameter to be measured. In particular, the first period of measurement time should be a time, which is longer than a calibration phase of the medical imaging apparatus (or of the components thereof). In a calibration phase the medical imaging apparatus may be tested and first data sets for each respective parameter may be measured and stored. For a precise maintenance prediction, it may be necessary that the parameter may be measured during different operation modes of the medical imaging apparatus, such that the different operation modes can be identified and the respective values of the parameter relating to the each operation mode may be identified. Therefore, a short time data set acquisition, as may happen in the calibration phase, would not provide sufficient information about the parameter values, parameter signatures in the operation modes.

According to an exemplary embodiment of the invention, the method may further comprise that the one or more trigger points may be at least one of a predefined rotation speed of a component of the medical imaging apparatus, a predefined rotation speed of the medical imaging apparatus, a start-up of the medical imaging apparatus, a defined angle position of the medical imaging apparatus, i.e. an X-ray tube, with respect to a gantry, a determined electrode beam focus of a component of the medical imaging apparatus, a load exerted on a component of the medical imaging apparatus, a load exerted on the medical imaging apparatus, a predefined temperature of a component of the medical imaging apparatus, a predefined temperature of the medical imaging apparatus, a defined temperature range of the medical imaging apparatus, a predefined operation time of a component of the medical imaging apparatus, and a predefined operation time of the medical imaging apparatus. This list of trigger points may not be limiting, also trigger points equivalent to the parameter described in embodiments hereinabove may be used.

According to an exemplary embodiment of the invention, the method may further comprise when the step of validating is completed, identifying changes of the parameter of the second data set, and if changes are identified the feedback signal provides an estimation of a maintenance based on the changes of the parameter of the second data set. The estimation may be based on the amount of difference of the parameter(s) of the data sets, wherein the allowed amount may be based on the component, which is monitored, and/or on the parameter, which should be measured.

According to an exemplary embodiment of the invention, the method may further comprise, if the data sets of the respective operation modes differ from each other determining the parameter of difference from the data set and linking the parameter of difference to a respective component of the medical imaging device for maintenance prediction of the respective component. Further, the step of linking comprises linking the parameter to the position of the component in the medical imaging apparatus and/or geometrical position of the medical imaging apparatus with respect to a medical imaging system for operating the medical imaging device. For instance, an X-ray tube may be in the 0° position or in the 90° position in a CT-gantry, wherein forces on a bearing of the X-ray tube are different and may depend also on the initial balancing quality of the bearing. Therefore, bearing aging effects could be detected for the two different positions and correlated with the initial data of manufacturing in the exact same positions.

According to an exemplary embodiment of the invention, the method may further comprise that the step of analyzing the second data set comprises analyzing the second data set thereby determining a plurality of operation modes of the medical imaging apparatus. The plurality of operation modes of the second data set are compared with the plurality of operation modes of the first data set thereby identifying from the plurality of operation modes of the first data set the operation modes which corresponds to the operation modes determined from the second data set by the data processing unit.

According to an exemplary embodiment of the invention, the method may further comprise wherein the step of analyzing comprises applying a machine learning algorithm and/or an artificial intelligence method to the first data set wherein the machine learning algorithm (and/or the artificial intelligence method) determines the plurality of operation modes.

According to an exemplary embodiment of the invention, the method may further comprise measuring a first data set of a plurality of parameters of the medical imaging apparatus by a plurality of sensors. Further, the method may comprise the step of measuring a second data set of a plurality of parameters of the medical imaging apparatus by a plurality of sensors, wherein for each component of the medical imaging apparatus a respective sensor is provided. The method may further comprise measuring a plurality of data sets of at least one parameter or of a plurality of parameters, wherein for each data set at least one operation modus of the medical imaging apparatus is determined, and/or measuring at least one digital signal of the data processing unit by at least one sensor thereby determining a status of the data processing of the data processing unit. For instance, the measurement of a digital signal may refer to a measurement of a control signal used for controlling the sensor and /or the data acquisition, hence the data processing unit. For preventing failures of data transmission and data processing, these digital signals may be measured and monitored, such that a replacement and/or repair of the component generating a digital signal can be predicted.

According to an exemplary embodiment of the invention, the method may further comprise, that the predetermined second period of measurement time may depend on the selected trigger point and the parameter to be measured. The selected trigger point may be preferably a start point of the predetermined second period of measurement time and/or wherein a selected range of the selected trigger point is preferably at least one of a length of the predetermined second period of measurement time, and a drift over the predetermined second period of measurement time. For instance, as a second period of measurement time an average value over at least a day, a week, or two weeks may be measured. Optionally, the second period of time may be a continuously measurement time or a sequentially measurement time. For example, for the temperature parameter a measurement period of a day may be sufficient, wherein for the rotation parameter a measurement period of a week is necessary for being able to determine any changes in the measured data set.

According to a further aspect of the present invention, a monitoring device used for maintenance prediction for a medical imaging apparatus is described. The device comprises at least one sensor configured for measuring a first data set of at least one parameter of the medical imaging apparatus over a predetermined first period of measurement time and measuring a second data set of the at least one parameter by the at least one sensor over a predetermined second period of measurement time. The device further comprises a data processing unit configured for analysing the first data set of the at least one parameter, thereby determining a plurality of operation modes of the medical imaging apparatus based on the measured parameter, and analysing the second data set thereby determining at least one operation modus in the second data set. The data processing unit is further configured for comparing the one operation modus determined from the second data set with the plurality of operation modes of the first data set for identifying from the plurality of operation modes of the first data set the operation modus, which corresponds to the operation modus, determined from the second data set. Further, it is configured for validating whether values of the measured parameter of the first and second data set differ from each other, and for providing a feedback signal based on the result of the validation, wherein the feedback signal is embodied as a maintenance prediction information.

According to an exemplary embodiment of the invention, the monitoring device further comprises for each component of the medical imaging apparatus at least one sensor provided for measuring a parameter of the component of the medical imaging apparatus, wherein the component of the medical imaging apparatus to which the sensor is attached is at least one of a motor, an emitter, a vacuum, a housing, a cooling medium, a cathode, an anode, a rotor, a stator. This list is to which component the sensor may be attached is not limiting, also other locations may be preferred depending on the parameter to be measured. Therefore, the monitoring device may comprise a plurality of sensors configured for measuring a data set and/or a plurality of data sets of the medical imaging apparatus.

According to an exemplary embodiment of the invention, the monitoring further comprises an indicating unit configured for indicating an optical and/or acoustical signal, which may preferably be the feedback signal, based on the result of the validation. The indicating unit may also be a signal to system control unit and the medical imaging apparatus (system) may indicate the signal and/or may be a remote signal via wired, and/or may be a wireless interface to external signal indication unit.

According to an exemplary embodiment of the invention, the data processing unit is configured for storing the data received from the at least one sensor and/or is configured for processing the stored data. The monitoring device may comprise a storage unit configured for storing the data received from the sensor, wherein the storage unit may be part of the data processing unit or may be an extra part, or a (wireless) data transmission to a cloud like storage. Further, the data may be stored on an external unit, like any processing unit capable for storing data, for further processing. The data may be stored or may not be stored and/or processed data may be stored or may not be stored, wherein it can be decided whether the data should be available for later examination, data comparison, calibration of the sensor, calibration of the medical imaging apparatus, and/or failure analysis. For instance, each data set, data sub-sets, and/or each data sample may be stored, wherein only the matching data sets, sub-sets, and data samples may be stored, such that later on only the matching data is used for further processing. This may be used as a data reduction method and help for selecting the relevant data for analysis and may be used to store only results and/or intermediate results.

According to a further aspect of the invention, a component, for a medical imaging apparatus, comprises a monitoring device according to any of the described embodiments and wherein the monitoring device is configured to perform the method as described with the respective embodiments. The monitoring device is arranged at the component, and/or wherein the monitoring device is removable arranged at the component. For instance, the component may be a substantial functional device of the medical imaging apparatus. Further, the component is preferably an X-ray tube. According to this embodiment, the monitoring device may be used for monitoring at least parts of the medical imaging apparatus, i.e. the component. In particular, the component may be monitored itself or may be monitored as a part of the medical imaging apparatus. The component may be monitored during the operation modes of the medical imaging apparatus, such that the residual lifetime and/or a maintenance prediction of the component could be carried out. On the other hand, the operation mode of the component itself may be monitored, wherein the component is not a part of the medical imaging apparatus and later on the operation mode of the component comprises operation modes wherein the component is a functional part of the medical imaging apparatus. The described embodiments referring only to a medical imaging apparatus may not only be limited the medical imaging apparatus, they may also be applicable for a component of the medical imaging apparatus.

According to a further aspect of the invention, a medical imaging apparatus, a monitoring device according to any of the above described embodiments, wherein the monitoring device is configured to perform the method as described with the respective embodiments. The medical imaging apparatus is preferably a medical X-ray imaging apparatus and/or wherein the monitoring device is arranged at the medical imaging apparatus, and/or wherein the monitoring device is removable arranged at the medical imaging apparatus. On the other hand, the medical imaging apparatus may also be a MRT, PET, SPECT, US or other medical imaging devices may be monitored. Further, the medical imaging apparatus may comprise a medical imaging unit for generating a medical imaging of a patient.

According to a further aspect of the invention, the step of analysing the data sets may comprise applying a machine learning algorithm and/or artificial intelligence methods for identification of the operation mode. Both the machine learning algorithm and the AI methods may be applied to the measured parameter(s), the determined sensor conditions, and/or the determined operation mode for comparison with a previously determined operation mode. Hence, these algorithm and methods may be used for lifetime prediction and maintenance prediction of the medical imaging apparatus or a component thereof. For a precise prediction of the maintenance it is important to get ground truth data and other relevant information about the key components that are under inspection, therefore the monitoring device is used.

According to a further aspect of the invention, a computer program element for evaluating a functionality of a medical imaging apparatus is presented, wherein the computer program element, when being executed by a processor of a monitoring device as presented herein, is adapted to cause the monitoring device to measure measure a first data set of at least one parameter of the medical imaging apparatus by at least one sensor of the monitoring device. The measuring of the first data set is performed over a predetermined first period of measurement time. Further, the monitoring device is caused to analyse, by a data processing unit, the first data set thereby determining a plurality of operation modes, in which the medical imaging apparatus was during the measurement time, based on the measured parameter. Furthermore, it is caused to measure at least a second data set of the at least one parameter by the at least one sensor o the monitoring device, wherein the measuring of the second data set is performed over a predetermined second period of measurement time. Moreover, the monitoring device is caused by the computer program element to analyse, by the data processing unit, the second data set thereby determining at least one operation modus, in which the medical imaging apparatus was during the second measurement time, and to compare the operation modus determined from the second data set with the plurality of operation modes of the first data set by the data processing unit, thereby identifying from the plurality of operation modes of the first data set the operation modus which corresponds to the operation modus determined from the second data set by the data processing unit. The monitoring device, is further caused to validate, by the data processing unit, whether values of the measured parameter of the first and second data set differ from each other, and caused to generate a feedback signal based on the result of the validation by the data processing unit, wherein the feedback signal is embodied as a maintenance prediction information for a user of the medical imaging apparatus.

The computer program element may be part of a computer program, but it can also be an entire program by itself. For example, the computer program element may be used to update an already existing computer program to get to the present invention.

The program element may be stored on a computer readable medium. The computer readable medium may be seen as a storage medium, such as for example, a USB stick, a CD, a DVD, a data storage device, a hard disk, or any other medium on which a program element as described above can be stored.

### BRIEF DESCRIPTION OF THE DRAWINGS

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.
Fig. 1 illustrates schematically a method of maintenance prediction for a medical imaging apparatus according to an exemplary embodiment of the invention.
Fig. 2 illustrates a signal signature of a data set according to an exemplary embodiment of the inventing.
Fig. 3 illustrates a further signal signature of a data set according to an exemplary embodiment of the inventing
Fig 4 illustrates schematically a monitoring device according to an exemplary embodiment of the invention.

### DETAILED DESCRIPTION OF EMBODIMENTS

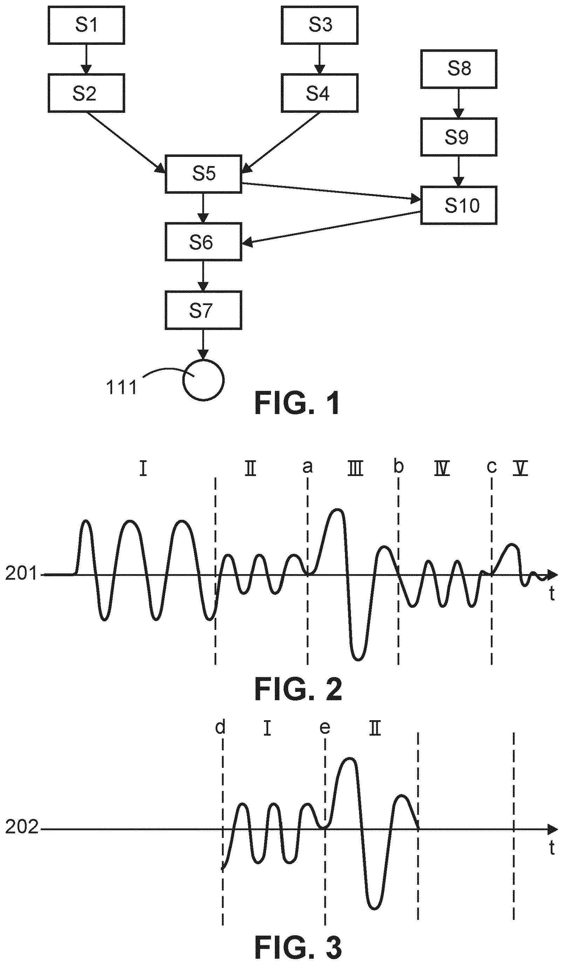

Fig. 1 illustrates a flow diagram of a method of prediction for a medical imaging apparatus according to an exemplary embodiment of the present invention. The method comprising the steps of measuring S1 a first data 201 set of at least one parameter of the medical imaging apparatus by at least one sensor, wherein the measuring of the first data set is performed over a predetermined first period of measurement time. The method further comprises the step of analyzing S2, by a data processing unit, the first data 201 set thereby determining a plurality of operation modes, in which the medical imaging apparatus was during the measurement time, based on the measured parameter. Further comprising the step of measuring S3 at least a second data set 202 of the at least one parameter by the at least one sensor, wherein the measuring of the second data set is performed over a predetermined second period of measurement time. Furthermore, the method comprises the step of analyzing S4, by the data processing unit, the second data set 202 thereby determining at least one operation modus, in which the medical imaging apparatus was during the second measurement time, and comparing the operation modus S5 determined from the second data set with the plurality of operation modes of the first data set by the data processing unit thereby identifying from the plurality of operation modes of the first data set the operation modus which corresponds to the operation modus determined from the second data set by the data processing unit. After the step S5 a validating step S6 is performed, by the data processing unit, validating whether values of the measured parameter of the first and second data set differ from each other, and generating S7 a feedback signal 111 based on the result of the validation by the data processing unit. The feedback signal 111 may be embodied as a maintenance prediction information for a user of the medical imaging apparatus and may be outputted afterwards by an indication unit to the user. Moreover, the method may comprise a measuring step S8 of a further (third or more) data set which measurement may be equal to the measurement of step S3. After measuring the further data set in step S8 the method may perform step S9 which comprises analyzing the further data set, wherein this step may be carried out equivalent to step S4. In step S10 the analyzed further data set may be compared with the first data set and/or it may be compared with second data set. The method steps S8 to S 10 may be repeated for a non-limiting amount of data sets, wherein the data set analyzed and compared in step S10 may be compared with the first data set and/or any other previously analyzed data set. After the comparison of a data set they will be processed to a validation step S6 and a generation step S7 of the method.

Fig. 2 illustrates a signal signature of a data set of a medical imaging apparatus according to an exemplary embodiment of the present invention. In particular, a first measured data set 201 is illustrated measured over a first period of measurement time t. The illustration in Fig. 2 is only schematically and should not be referred to any specific measured parameter, this illustration should only indicate a possible signal signature of a measured data set. The first data set 201 may be measured for at least five different operation modes I to V. As can be seen in Fig. 2, in each operation mode the signature, the curve over the time, differs from each other. Preferably, the first data set 201 comprises all possible operation modes of the medical imaging apparatus, such that when compared with other measured data sets, the respective equal or similar operation modes in other data set may be determined.

Fig. 3 illustrates a further signal signature of a further data set of a medical imaging apparatus according to an exemplary embodiment of the present invention. In particular, a first measured data set 202 is illustrated and is measured over a second period of measurement time t. As can be seen in comparison with Fig. 2, the second period of measurement time is shorter that the first period of measurement time. When performing the step of comparing both data sets, the result may indicate that the second data set 202 comprises two operations modes I and II, which are similar to operation modes II and III of the first data set illustrated in Fig. 2. Points d and e in Fig. 3 and a to c in Fig. 2 respectively may indicate trigger points at which data samples may be selected. For instance, trigger point b may indicate a change of a positive signal to a negative signal (value), trigger point c may indicate a start of a signal form a zero curve to an increasing signal curve, wherein trigger point e may indicate a decrease of a signal curve (value). The respective trigger points for the data samples of the data set may be chosen by the user depending on the component and/or the parameter, which should be monitored, i.e. for which a maintenance prediction should be carried out.

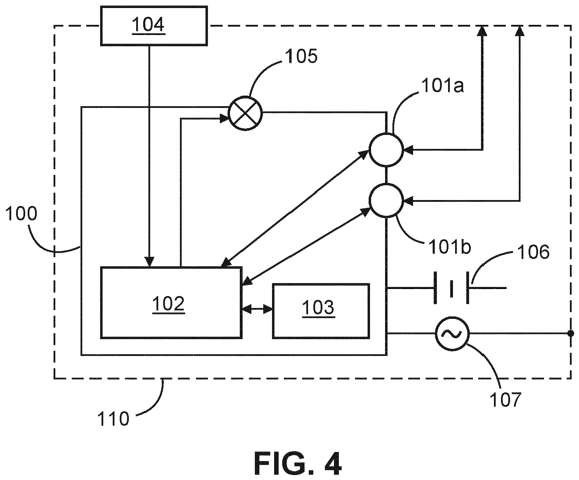

Fig. 4 illustrates a monitoring device 100 for maintenance prediction for a medical imaging apparatus 110 according to an exemplary embodiment of the present invention. The monitoring device 100 comprises at least one sensor 101 configured for measuring a first data set of at least one parameter of the medical imaging apparatus 110 over a predetermined first period of measurement time and measuring a second data set of the at least one parameter by the at least one sensor over a predetermined second period of measurement time. Further, it comprises a data processing unit 102 configured for analysing the first data set of the at least one parameter, thereby determining a plurality of operation modes of the medical imaging apparatus 110 based on the measured parameter, and configured for analysing the second data set thereby determining at least one operation modus in the second data set. Further, the data processing unit 102 may be configured for comparing the one operation modus determined from the second data set with the plurality of operation modes of the first data set for identifying from the plurality of operation modes of the first data set the operation modus, which corresponds to the operation modus, determined from the second data set. Further, it may be configured for validating whether values of the measured parameter of the first and second data set differ from each other, and for providing a feedback signal based on the result of the validation, wherein the feedback signal is embodied as a maintenance prediction information. The data processing unit 102 is configured for controlling the at least one sensor 101. As can be seen in Fig. 4 the illustrated monitoring device comprises at least two sensors 101a and 101b for measuring a parameter of the medical imaging apparatus 110. For example, the sensors 101a, 101b may be used for measuring the same and/or different parameters of the medical imaging apparatus 110. The medical imaging apparatus 110 is illustrated as a dotted line because the monitoring device 100 may be integrated in the medical imaging apparatus 110 or not. The data processing unit may be configured for controlling the sensors 101a, 101b, an indicating unit 105 and a storage unit 103. Further, the data processing unit 102 may be configured for storing the data received from the at least one sensor 101 and/or is configured for processing the stored data for at least one of the different data sets or preferably for all data sets of the medical imaging apparatus 110. The monitoring device 100 may comprise the storage unit 103 configured for storing the data received from the sensor 101, wherein the storage unit 103 is illustrated in Fig. 4 as a part of the monitoring device. Contrary, the storage unit 103 may be part of the data processing unit 102 or may be an extra external part, or may be cloud storage via an interface connection. The indicating unit 105 may be configured for indicating an optical and/or acoustical signal as a feedback signal based on the result of the validation of the data sets, wherein the signal is indicated to a user operating the monitoring device and/or the medical imaging apparatus. The indicating unit 105 may also be a signal to system control unit and the medical imaging apparatus (system) may indicate the signal and/or may be a remote signal via wired, and/or may be a wireless interface to external signal indication unit. Additionally, the data processing unit 102 may receive from an external trigger unit 104 respective signals, which may indicate the start or the end of an operation mode. The monitoring device 100 in Fig. 4 comprises a self-sustainable power supply 106 and may additionally be connected to the power supply 107 of the medical imaging apparatus, wherein the power supplies 106, 107 may interchangeable with each other.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 100: monitoring device
- 101a,b: sensor
- 102: data processing unit
- 103: storage unit
- 104: trigger unit
- 105: indicating unit
- 106: self-sustainable power supply
- 107: power supply
- 110: medical imaging apparatus
- 111: feedback signal
- 201: first data set
- 202: second data set
- I-V: operation modus
- t: time
- a-e: trigger points

## Claims

1. Method of maintenance prediction for a medical imaging apparatus, the method comprising the steps of
measuring (S1) a first data (201) set of at least one parameter of the medical imaging apparatus by at least one sensor, wherein the measuring of the first data set is performed over a predetermined first period of measurement time,
analysing (S2), by a data processing unit, the first data (201) set thereby determining a plurality of operation modes, in which the medical imaging apparatus was during the measurement time, based on the measured parameter,
measuring (S3) at least a second data set (202) of the at least one parameter by the at least one sensor, wherein the measuring of the second data set is performed over a predetermined second period of measurement time,
analysing (S4), by the data processing unit, the second data set (202) thereby determining at least one operation modus, in which the medical imaging apparatus was during the second measurement time,
comparing the operation modus (S5) determined from the second data set with the plurality of operation modes of the first data set by the data processing unit thereby identifying from the plurality of operation modes of the first data set the operation modus which corresponds to the operation modus determined from the second data set by the data processing unit,
validating (S6), by the data processing unit, whether values of the measured parameter of the first and second data set differ from each other, and
generating (S7) a feedback signal based on the result of the validation by the data processing unit, wherein the feedback signal is embodied as a maintenance prediction information for a user of the medical imaging apparatus.

2. Method according to claim 1,
wherein the measured parameter is at least one of a vibration of a component of the medical imaging apparatus, a vibration of the medical imaging apparatus, an acceleration of a component of the medical imaging apparatus, an acceleration of the medical imaging apparatus, a surrounding temperature of a component of the medical imaging apparatus, a surrounding temperature of the medical imaging apparatus, a surrounding humidity of a component of the medical imaging apparatus, a surrounding humidity of the medical imaging apparatus, a temperature of the medical imaging apparatus, a temperature of a component of the medical imaging apparatus, a mechanical shock of the medical imaging apparatus, a mechanical shock of a component of the medical imaging apparatus, a vacuum parameter of the medical imaging apparatus, an emitter parameter of the medical imaging apparatus, a power supply parameter of the medical imaging apparatus, an over/under voltage parameter of a component of the medical imaging apparatus, an over/under voltage parameter of the medical imaging apparatus, an over/under current parameter of a component of the medical imaging apparatus, an over/under current parameter of the medical imaging apparatus, a X-ray radiation parameter of the medical imaging apparatus, a rotation speed of a component of the medical imaging of the apparatus relative to the medical imaging apparatus, a rotation speed of the medical imaging apparatus, an electron beam parameter of the medical imaging apparatus, and a magnetic field parameter of the medical imaging apparatus.

3. Method according to claim 1 or 2,
selecting data samples, at one or more predefined trigger points, of the second data set, and wherein the step of analysing the second data set comprises only the analysis of the selected data samples, and
comparing, by the data processing unit, the data samples selected from the second data set with the plurality of operation modes of the first data set thereby identifying, by the data processing unit, from the plurality of operation modes of the first data set the operation modus which corresponds to the data samples determined from the second data set,
wherein the step of validating comprises validating whether values of the measured parameter of the compared data samples of the second data set and the operation modes of the first data set differ from each other.

4. Method according to any of the preceding claims,
wherein the predetermined first period of measurement time corresponds to an active operating life of the medical imaging apparatus,
wherein the active operating life of the medical imaging apparatus is one week of active operation life of the medical imaging apparatus.

5. Method according to claim 3,
wherein the one or more trigger points is at least one of a predefined rotation speed of a component of the medical imaging apparatus, a predefined rotation speed of the medical imaging apparatus, a start-up of the medical imaging apparatus, a defined angle position of the medical imaging apparatus, i.e. an X-ray tube, with respect to a gantry, a determined electrode beam focus of a component of the medical imaging apparatus, a load exerted on a component of the medical imaging apparatus, a load exerted on the medical imaging apparatus, a predefined temperature of a component of the medical imaging apparatus, a predefined temperature of the medical imaging apparatus, a defined temperature range of the medical imaging apparatus, a predefined operation time of a component of the medical imaging apparatus, and a predefined operation time of the medical imaging apparatus.

6. Method according to any one of the preceding claims, further comprising
wherein when the step of validating is completed, identifying changes of the parameter of the second data set, and if changes are identified the feedback signal provides an estimation of a maintenance based on the changes of the parameter of the second data set.

7. Method according to any one of the preceding claims, wherein the method further comprises,
if the data sets of the respective operation modes differ from each other determining the parameter of difference from the data set and linking the parameter of difference to a respective component of the medical imaging device for maintenance prediction of the respective component,
wherein the step of linking comprises linking the parameter to the position of the component in the medical imaging apparatus and/or geometrical position of the medical imaging apparatus with respect to a medical imaging system for operating the medical imaging device.

8. Method according to any one of the preceding claims,
wherein the step of analysing (S4) the second data set comprises analysing the second data set thereby determining a plurality of operation modes of the medical imaging apparatus,
wherein the plurality of operation modes of the second data set are compared with the plurality of operation modes of the first data set thereby identifying from the plurality of operation modes of the first data set the operation modes which corresponds to the operation modes determined from the second data set by the data processing unit.

9. Method according to any one of the preceding claims,
wherein the step of analysing comprises applying a machine learning algorithm to the first data set wherein the machine learning algorithm determines the plurality of operation modes.

10. Method according to any one of the preceding claims, wherein the method further comprises
measuring a first data set of a plurality of parameters of the medical imaging apparatus by a plurality of sensors, and/or
measuring a second data set of a plurality of parameters of the medical imaging apparatus by a plurality of sensors,
wherein for each component of the medical imaging apparatus a respective sensor is provided, and/or the method further comprises
measuring a plurality of data sets of at least one parameter or of a plurality of parameters, wherein for each data set at least one operation modus of the medical imaging apparatus is determined, and/or
measuring at least one digital signal of the data processing unit by at least one sensor thereby determining a status of the data processing of the data processing unit.

11. Method according to any one of the preceding claims,
wherein the predetermined second period of measurement time depends on the selected trigger point and the parameter to be measured,
wherein the selected trigger point is preferably a start point of the predetermined second period of measurement time and/or wherein a selected range of the selected trigger point is preferably at least one of a length of the predetermined second period of measurement time, and a drift over the predetermined second period of measurement time.

12. A monitoring device (100) for maintenance prediction for a medical imaging apparatus (110), the device comprising
at least one sensor (101) configured for measuring a first data set of at least one parameter of the medical imaging apparatus over a predetermined first period of measurement time and measuring a second data set of the at least one parameter by the at least one sensor over a predetermined second period of measurement time,
a data processing unit (102) configured for
analysing the first data set of the at least one parameter, thereby determining a plurality of operation modes of the medical imaging apparatus based on the measured parameter,
analysing the second data set thereby determining at least one operation modus in the second data set,
comparing the one operation modus determined from the second data set with the plurality of operation modes of the first data set for identifying from the plurality of operation modes of the first data set the operation modus which corresponds to the operation modus determined from the second data set,
validating whether values of the measured parameter of the first and second data set differ from each other,
providing a feedback signal based on the result of the validation,
wherein the feedback signal is embodied as a maintenance prediction information.

13. The monitoring device according to claim 12,
wherein for each component of the medical imaging apparatus at least one sensor (101) provided for measuring a parameter of the component of the medical imaging apparatus,
wherein the component of the medical imaging apparatus to which the sensor is attached is at least one of a motor, an emitter, a vacuum, a housing, a cooling medium, a cathode, an anode, a rotor, a stator.

14. A medical imaging apparatus comprising,
a monitoring device (100) according to any one of the claims 12 or 13,
wherein the medical imaging apparatus is preferably an X-ray imaging device, more preferably an X-ray tube.

15. A computer program element for maintenance prediction for a medical imaging apparatus,
wherein the computer program element, when being executed by a processor of a monitoring device, is adapted to cause the monitoring device to
measure a first data set of at least one parameter of the medical imaging apparatus by at least one sensor of the monitoring device, wherein the measuring of the first data set is performed over a predetermined first period of measurement time
analyse, by a data processing unit, the first data set thereby determining a plurality of operation modes, in which the medical imaging apparatus was during the measurement time, based on the measured parameter,
measure at least a second data set of the at least one parameter by the at least one sensor o the monitoring device, wherein the measuring of the second data set is performed over a predetermined second period of measurement time,
analyse, by the data processing unit, the second data set thereby determining at least one operation modus, in which the medical imaging apparatus was during the second measurement time,
compare the operation modus determined from the second data set with the plurality of operation modes of the first data set by the data processing unit, thereby identifying from the plurality of operation modes of the first data set the operation modus which corresponds to the operation modus determined from the second data set by the data processing unit,
validate, by the data processing unit, whether values of the measured parameter of the first and second data set differ from each other, and
generate a feedback signal based on the result of the validation by the data processing unit, wherein the feedback signal is embodied as a maintenance prediction information for a user of the medical imaging apparatus.
